(19) Europäisches Patentamt
European Patent Office
Office européen des brevets

(11) Veröffentlichungsnummer: **0 157 738**
**B1**

(12) **EUROPÄISCHE PATENTSCHRIFT**

(45) Veröffentlichungstag der Patentschrift: **19.04.89**

(21) Anmeldenummer: **85810115.7**

(22) Anmeldetag: **18.03.85**

(51) Int. Cl.⁴: **C 07 B 61/02** //
C07D211/94, C07D263/04,
C07D263/52, C07D241/08,
C07D401/04, C07D498/10

# (54) Verfahren zur Herstellung von Nitroxylen sterisch gehinderter Amine.

(30) Priorität: **22.03.84 US 592317**

(43) Veröffentlichungstag der Anmeldung: **09.10.85 Patentblatt 85/41**

(45) Bekanntmachung des Hinweises auf die Patenterteilung: **19.04.89 Patentblatt 89/16**

(84) Benannte Vertragsstaaten: **DE FR GB IT NL**

(56) Entgegenhaltungen:
**US-A-3 634 346**

**COLLECTION OF CZECHOSLOVAK CHEMICAL COMMUNICATIONS, Band 46, Nr. 5, 1981, Seiten 1071-1082; V. CHOLVAD et al.: "Hydrogen and electron transfer from diphenylamine in reaction with coordinated peroxy-radicals and hydroperoxides"**

(73) Patentinhaber: **CIBA- GEIGY AG, Klybeckstrasse 141, CH- 4002 Basel (CH)**

(72) Erfinder: **Winter, Roland A. E., 23 Banksville Road, Armonk New York 10504 (US)**
Erfinder: **Malherbe, Roger F., Dr., Bleichestrasse 7, CH- 4058 Basel (CH)**

**EP 0 157 738 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Nitroxyle (auch N-Oxyle oder Nitroxide genannt) sind freie Radikale mit einem ungepaarten Elektron. Sie werden durch Oxidation sekundärer Amine erhalten und sind stabile Verbindungen, wenn die beiden mit dem N-Atom verbundenen C-Atome keinen Wasserstoff tragen, d.h. tertiäre C-Atome sind. Für ihre Herstellung aus sekundären Aminen kann als Oxydationsmittel z. B. Ozon verwendet werden (S.D Razumovskii et al., Dokl. Akad. Nauk. SSSR 183, 1106 (1969), ref. Chem. Abstracts 70 (1969), 95987) oder einer Percarbonsäure (G. Chapelet-Letourneux et al., Bull. Soc. Chim. 1965, 3283) oder wässriges Wasserstoffperoxid in Gegenwart von Perwolframat-Ionen (O.L. Lebedew et al., Dokl. Akad. Nauk. SSSR 140, 1327 (1961), Proc. Acad. Sci. USSR, Chem.Sect. 140, 1046 (1962); O.L. Lebedew et al., Chem. Abstracts 56 (1962), 15479).

Letztere Methode hat sich gut bewährt für die oxydation wasserlöslicher sekundärer Amine. Wenn das Amin jedoch wenig löslich in Wasser ist, wird die Reaktion mit wässrigem $H_2O_2$ jedoch sehr langsam und die Ausbeuten sind niedrig.

Persäuren, wie z. B. m-Chlorperbenzoesaure,können in organischen Lösungsmitteln, wie z. B. Methylenchlorid eingesetzt werden, sind aber teure Reagentien und können Störungen durch Salzbildung mit dem Amin geben.

Ein neutrales Oxidationsmittel, das in organischer Lösung verwendet werden kann, sind organische Hydroperoxide, wie z. B. t-Butylhydroperoxid. O.W. Maender und E.G. Janzen/J. Org. Chem. 34 (1969), 4082 haben versucht, t-Butylhydroperoxid zur Oxydation von sekundären Tritylaminen zu den entsprechend Nitroxylen zu verwenden, fanden dieses Reagens jedoch als ungeeignet. Einige Jahre später wurde im US-Patent 3 634 346 gezeigt, dass man cyclische sekundäre Amine mittels organischen Hydroperoxiden in Gegenwart von Metallsalz-Katalysatoren zu den entsprechenden Lactamen oxydieren kann. Daraus war zu schliessen, dass die Reaktion sekundärer Amine mit organischen Hydroperoxiden nicht zur Bildung von Nitroxylen führt.

Es war daher überraschend zu finden, dass man durch Einwirkung von organischen Hydroperoxiden auf cyclische sekundäre Amine, die in α-Stellung am N-Atom tertiäre C-Atome besitzen, in Gegenwart bestimmter Metallverbindungen als Katalysatoren die entsprechenden Nitroxyle in hoher Ausbeute und Reinheit erhält.

Die Erfindung betrifft daher ein Verfahren zur Herstellung von Nitroxylen der Formel I,

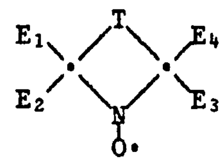

(I)

worin $E_1$ und $E_3$ unabhängig voneinander $C_1$-$C_5$-Alkyl oder Phenyl sind, $E_2$ und $E_4$ unabhängig voneinander $C_1$-$C_5$-Alkyl sind oder $E_1$ und $E_2$ zusammen oder $E_3$ und $E_4$ zusammen Tetramethylen oder Pentamethylen sind und T eine zweiwertige Gruppe ist, die zusammen mit dem Stickstoffatom und den beiden tertiären Kohlenstoffatomen einen 5- oder 6-gliedrigen Ring bildet, durch Umsetzung eines cyclischen sekundären Amines der Formel II

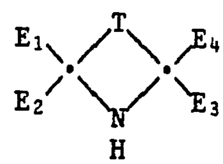

(II)

in einem inerten organischen Lösungsmittel mit einem organischen Hydroperoxid in Gegenwart katalytischer Mengen eines Metallcarbonyls, Metalloxides, Metallacetylacetonates oder Metallalkoxides eines Metalles der Gruppen IVB, VB, VIB, VIIB oder VIII des periodischen Systems, bei einer Temperatur von 0 bis 200° C und bei einem Molverhältnis Hydroperoxid: Amin von 50 : 1 bis 1 : 10.

Vorzugsweise sind $E_1$, $E_2$, $E_3$ und $E_4$ Methyl. Die Natur der Gruppe T ist nicht kritisch, sie kann verschiedene Arten von Substituenten enthalten, soferne diese unter den Reaktionsbedingungen inert gegenüber Hydroperoxiden sind.

Beispiele für Verbindungen der Formel I, die erfindungsgemäss hergestellt werden können, sind 4-Hydroxy-2,2,6,6-tetramethylpiperidin-1-oxyl, 3°-Carbamoyl-2,2,5,5-tetramethylpyrrolidin-1-oxyl, N-(1-Oxyl-2,2,6,6-tetramethylpiperidin-4-yl)-ε-caprolactam, 3-Oxyl-2,2,4,4-tetramethyl-7-oxa-3,20-diaza-spiro[5.1.11.2]-heneicosan-21-on, 4-Aza-3,3-dimethyl-4-oxyl-1-oxa-spiro[4.5]decan oder 2,4,4,-Trimethyl-2-phenyloxazolidin-3-oxyl.

Die als Oxydationsmittel verwendeten organischen Hydroperoxide sind Verbindungen, in denen sich die Hydroperoxidgruppe -OOH an einem tertiären C-Atom befindet. Beispiele hierfür sind tert-Butylhydroperoxid, tert-Amylhydroperoxid, tert-Hexylhydroperoxid, tert-Octylhydroperoxid, Ethylbenzol-α-hydroperoxid, Tetralinhydroperoxid oder Cumol-α-hydroperoxid.

Bevorzugt verwendet man tert-Butylhydroperoxid, tert-Amylhydroperoxid, Ethylbenzol-α-hydroperoxid oder Cumolhydroperoxid als Oxydationsmittel. Besonders bevorzugt sind tert-Butyl- und Cumol-hydroperoxid.

Die Reaktion wird in flüssiger Phase ausgeführt

unter Verwendung eines inerten Lösungsmittels. Beispiele hierfür sind Ester, wie z. B. Butylacetat; Ketone, wie z. B. Aceton; Ether, wie z. B. Dibutylether, Dioxan oder Tetrahydrofuran; Chlorkohlenwasserstoffe, wie z. B. Methylenchlorid, 1,2-Dichlorethan, Chlorbenzol oder o-Dichlorbenzol; oder Kohlenwasserstoffe, wie z. B. Hexan, Decan, Benzol, Toluol, Xylol oder Cumol (Isopropylbenzol). Im allgemeinen verwendet man das Lösungsmittel in einer Menge von bis zu 20 Molen pro Mol Hydroperoxid.

Vorzugsweise wird das Amin, der Katalysator und das Lösungsmittel im Reaktionsgefäss vorgelegt und dazu wird unter Rühren und unter Kontrolle der Reaktionstemperatur eine Lösung des Hydroperoxides zugegeben. Man kann die Reaktion auch kontinuierlich ausführen, wobei man das Amin oder eine Lösung des Amines mit einer Lösung des Hydroperoxidea und des Katalyators in Kontakt bringt.

Die Reaktion kann bei Temperaturen von 0 bis 200°C ausgeführt werden, insbesondere bei 50 bis 150°C.

Beispiele für geeignete Katalysatoren sind Vanadylacetylacetonat, Cobaltcarbonyl, Titan(IV)-isopropoxid, $Mo(CO)_6$ oder $MoO_3$. Bevorzugt verwendet man Molybdän- und Titan-Katalysatoren.

Die verwendete Menge an Katalysator ist nicht kritisch. Im allgemeinen genügen kleine Mengen um die Reaktion zu initieren. Bevorzugt verwendet man 0,001 Mol-% bis 0,1 Mol-%, berechnet auf das Hydroperoxid.

Der Verlauf der Reaktion kann durch chromatographische oder spektroskopische Analysenmethoden verfolgt werden. Am Ende der Reaktion wird der Katalysator abgetrennt und das Produkt aus der Lösung in üblicher Weise isoliert, beispielsweise durch Eindampfen oder durch Kristallisation.

Man kann die Reaktionslösung aber auch ohne Isolierung des Nitroxyls einer Reduktion unterwerfen und erhält dann als Produkt die entsprechende N-Hydroxylverbindung, die man auch als ein cyclisches Hydroxylamin bezeichnen kann. Die Reduktion kann z. B. durch katalytische Hydrierung in Gegenwart von Edelmetall- oder Nickel-Katalysatoren bewirkt werden oder durch Umsetzung mit Boranen oder metallischem Zink oder anderen bekannten Reduktionsmitteln.

Die Erfindung betrifft daher auch ein Verfahren zur Herstellung von Verbindungen der Formel III;

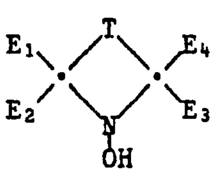

(III)

durch Umsetzung einer Verbindung der Formel II wie vorhin beschrieben und anschliessende reduktive Behandlung des Reaktionsgemisches ohne Isolierung des Nitroxyl-Zwischenproduktes der Formel I.

Die Verbindungen der Formel I sind orange bis rot gefärbte Verbindungen und können auf Grund ihres ungepaarten Elektrons als Spinmarkierung für die ESR-Spektroskopie verwendet werden. Sie können weiterhin als Inhibitoren der radikalischen Polymerisation ungesättigter Verbindungen sowie als Stabilisatoren für organische Polymere gegen thermischen und photochemischen Abbau verwendet weden.

Die Verbindungen der Formel III sind farblose Verbindungen und können als Antioxydantien for organische Materialien verwendet werden.

Die folgenden Beispiele beschreiben die Erfindung näher ohne ihren Umfang auf die Beispiele beschränken zu wollen. Darin sind alle Temperaturangaben in °C gegeben.

**Beispiel 1:**

Herstellung von 4-Hydroxy-2,2,6,6-tetramethyl-piperidin-1-oxyl

8,5 g (54 mMol) 2,2,6,6-Tetramethylpiperidin-4-ol und 0,1 g $Mo(CO)_6$ werden in 50 ml analysenreinem 1,2-Dichlorethan unter Erwärmen gelöst. Dazu werden 27 ml einer 4-molaren Lösung von tert-Butylhydroperoxid in 1,2-Dichlorethan so zugetropft, dass die Lösung auf Rückfluss-Temperatur bleibt. Anschliessend wird 4 h zum Rückfluss erhitzt.

Nach dieser Zeit zeigt eine gaschromatographische Analyse, dass die Reaktionslösung weniger als 2 % nichtumgesetztes Amin enthält. Die Reaktionslösung wird nach Abkühlen mit einer 5 %igen wässrigen Natriumsulfitlösung gewaschen. Die wässrige Phase wird mit 50 ml Chloroform extrahiert und die vereinigten organischen Phasen über $MgSO_4$ getrocknet.

Nach Eindampfen der Lösung wird der Rückstand aus Hexan kristallisiert und man erhält 8 g (= 86 % d. Th.) oranger Kristalle, die bei 69 - 71° schmelzen. H. Lemaire et al., Bull. Soc. Chim. France 1986, 886, geben für diese Verbindung einen Schmelzpunkt von 71° an.

**Beispiel 2:**

Wenn in obigem Beispiel das $Mo(CO)_6$ durch eine äquivalente Menge $MoO_3$ ersetzt wird, so sind nach 2-stündiger Reaktionszeit über 98 % des Piperidinols zum N-Oxyl umgesetzt entsprechend einer gaschromatographischen Analyse.

**Beispiel 3:**

Wenn im Beispiel 1 der $Mo(CO)_6$-Katalysator durch eine äquivalente Menge Vanadylacetylacetonat ersetzt wird, so verläuft die Reaktion langsamer und nach 2 Stunden sind nur 31 % des Piperidinols zum N-Oxyl umgesetzt.

**Beispiel 4:**

Herstellung von N-(1-oxyl-2,2,6,6-tetramethylpiperidin-4-yl)-ε-caprolactam

25,2 g (0,1 Mol) N-(2,2,6,6-Tetramethylpiperidin-4-yl)-ε-caprolactam und 0,2 g $Mo(CO)_6$ werden wie in Beispiel 1 beschrieben mit 50 ml einer 4-molaren Lösung von tert-Butylhydroperoxid umgesetzt und man erhält 26,6 g (= 99 % d. Th.) eines roten Feststoffes. Umkristallisation aus Hexan ergibt orange Kristalle, die bei 152 - 159° achmelzen.

| | | |
|---|---|---|
| Analyse ($C_{15}H_{27}N_2O_2$) | Ber. | C 67,3 % H 10,1 % N 10,4 % |
| | Gef. | C 66,9 % H 9,9 % N 12,2 % |

**Beispiel 5:**

Wenn in Beispiel 4 der $Mo(CO)_6$-Katalysator durch die äquivalente Menge $Ti(OC_3H_7\text{-iso})_4$ ersetzt wird, wobei das Molverhältnis Ti: Amin 0,04 beträgt, so zeigt eine gaschromatographische Analyse nach 5 h eine Umsetzung von 95 %.

**Beispiel 6:**

Herstellung von 4-Benzoyloxy-2,2,6,6-tetramethyl piperidin-1-oxyl

13,1 g (50 mMol)-4-Benzoyloxy-2,2,6,6-tetramethylpiperidin wird wie in Beispiel 1 beschrieben mit tert-Butylhydroperoxid in Dichlorethan in Gegenwart von $Mo(CO)_6$ oxydiert und man erhält bei analoger Verfahrensweise 9,5 g orange Kristalle, die nach Kristallisation aus Methanol bei 104-106° schmelzen. V.A. Golubev et al., Chem. Abstracts 64 (1966), 11164, geben für diese Verbindung einen Schmelzpunkt von 105° an.

**Beispiel 7:**

Herstellung von Di-(1-hydroxyl-2,2,6,6-tetramethyl-piperidin-4-yl)-sebacat

Eine Lösung von 15 g (31,2 mMol) Di-(2,2,6,6-tetramethylpiperidin-4-yl)-sebacat und 0,2 g (0,76 mMol) Molybdänhexacarbonyl in 100 ml 1,2-Dichlorethan wird zum Rückfluss erwärmt. 31,5 ml einer 4-molaren Lösung von tert-Butylhydroperoxid in 1,2-Dichlorethan (entsprechend 126 mMol Peroxid) wird innerhalb 15 Minuten zugegeben und die Lösung weitere 2,5 h zum Rückfluss erwärmt.

Nach Abkühlung wird die Lösung zweimal mit Wasser gewaschen und die oganische Phase in einen Hydrierkolben gefüllt. Dort wird die Lösung unter Zusatz einer kleinen Menge Palladium auf Aktivkohle bei Raumtemperatur und 2,8 bar $H_2$-Druck hydriert. Nach Abfiltrieren des Katalysators wird die Lösung eingedampft. Der feste Rückstand wird aus 250 ml Ethanol-Wasser 4: 1 unter Stickstoff-Atmosphäre umkristallisiert. Man erhält 13,6 g der 1-Hydroxylverbindung als farblose Verbindung, die bei 129-134° schmilzt. E. F Litvin et al., Chem. Abstracts 74 (1971), 64180, geben für diese Verbindung einen Schmelzpunkt von 101° an.

**Beispiel 8:**

Herstellung von 3-Oxyl-2,2,4,4-tetramethyl-7-oxa-3,20-diaza-spiro[5.1.11.2]heneicosan-21-on

Eine Lösung von 18,3 g 2,2,4,4-Tetramethyl-7-oxa-3,20-diaza-spiro[5.1.11.2]heneicosan-21-on in 100 ml 1,2-Dichlorethan werden mit 25 ml einer 4-molaren tert-Butylhydroperoxid-Lösung in Dichlorethan in Gegenwart von 0,3 g $Mo(CO)_6$ oxydiert. Nach 3 h Rückfluss zeigt ein Dünnschicht-Chromatogramm eine Umsetzung von über 95 %.

**Beispiel 9:**

Herstellung von 1,1'-Ethylen-bis(4-hydroxy-3,3,5,5-tetramethylpiperazin-2-on)

16,9 g (50 mMol) 1,1'-Ethylen-bis(3,3,5,5-tetramethylpiperazin-2-on) wird analog Beispiel 7 oxydiert und die Rektionslösung über Pd/C-Katalysator hydriert. Nach Abfiltrieren des Katalysators und Eindampfen der Dichlorethan-Lösung hinterbleiben 15,1 g eines hellrosafarbenen Rohproduktes, das bei 193° schmilzt. Umkristallisation aus Ethylacetat-Methanol gibt die farblose Titelverbindung vom Schmp. 200°C.

| | | |
|---|---|---|
| Analyse ($C_{18}H_{34}N_4O_4$) | Ber. | C 58,4 % H 9,3 % N 15,1 % |
| | Gef. | C 58,5 % H 9,3 % N 15,0 % |

NMR-Spektrum (DMSO-d6): 1,21 (s, 12 H, 4 $CH_3$ axial); 1,43 (s,12 H, 4 $CH_3$ equatorial); 3,20 und 3,50 (s,8 H, $CH_2N$); 4,60 (breites s, 2 H, OH).

**Beispiel 10:**

Herstellung von 4-Benzoyloxy-1-hydroxy-2,2,6,6-tetra-methylpiperidin

Eine Lösung von 26,2 g (0,1 Mol) 4-Benzoyloxy-2,2,6,6-tetramethylpiperidin und 0,2 g $Mo(CO)_6$ in 20 ml Toluol wird auf 60° erwärmt. 38,1 g einer 80 %-igen Lösung von Cumolhydroperoxid in Toluol (0,2 Mol) wird innerhalb 45 Minuten zugegeben wobei eine schwach exotherme Reaktion entsteht. Die rote Lösung wird dann weitere 30 Minuten auf 65° erwärmt und in eine Hydrierflasche gefüllt.

Nach Zufügen von 0,5 g Palladium/Aktivkohle-Katalysator wird die Lösung bei 3,5 bar $H_2$ 3 h hydriert. Der Katalysator wird dann abfiltriert und mit 100 ml Chloroform gewaschen. Das Filtrat wird eineengt bis alles Chloroform abgedampft ist. Dann werden 100 ml Hexan zugesetzt und die Lösung gekühlt. Man erhält 20 g weisser Kristalle, die bei 145 - 149° schmelzen.

V.A. Golubev et al., Chem. Abstracts 64 (1966), 11164, geben für diese Verbindung einen Schmelzpunkt von 135 - 146° an.

**Beispiel 11:**

Herstellung von 4-Aza-3,3-dimethyl-1-oxaspiro[4.5]decan-4-oxyl

Eine Lösung von 32,1 g (0,19 Mol) 4-Aza-3,3-dimethyl-1-oxaspiro[4.5]decan-1-oxyl und 0,7 g $Mo(CO)_6$ in 75 ml Toluol wird auf 90° erwärmt. 100 ml einer 4-molaren Lösung von tert-Butylhydroperoxid in Toluol werden innerhalb 30 Minuten zugegeben, anschliessend wird die Lösung weitere 45 Minuten auf 90° erwärmt. Man erhält die Titelverbindung, die beschrieben ist in J.Amer.Chem. Soc. 89 (1967), 3054.

**Beispiel 12:**

Herstellung von 2,4,4-Trimethyl-2-phenyloxazolidin-3-oxyl

Analog Beispiel 11 wird aus 2,4,4-Trimethyl-2-phenyloxazolidin das entsprechende N-Oxyl erhalten.

**Patentansprüche**

1. Verfahren zur Herstellung von Nitroxylen der Formel I

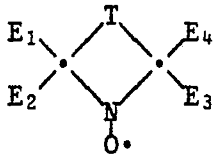

(I)

worin $E_1$ und $E_3$ unabhängig voneinander $C_1$-$C_5$-Alkyl oder Phenyl sind, $E_2$ und $E_4$ unabhängig voneinander $C_1$-$C_5$-Alkyl sind oder $E_1$ und $E_2$ zusammen oder $E_3$ und $E_4$ zusammen Tetramethylen oder Pentamethylen sind, und T eine zweiwertige Gruppe ist, die zusammen mit dem Stickstoffatom und den beiden tertiären Kohlenstoffatomen einen 5- oder 6-gliedrigen Ring bildet, durch Umsetzung eines cyclischen sekundären Amines der Formel II

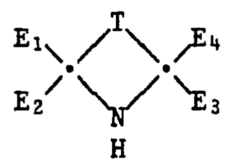

(II)

in einem inerten organischen Lösungsmittel mit einem organischen Hydroperoxid in Gegenwart von katalytischen Mengen eines Metallcarbonyls, Metalloxides, Metallacetylacetonates oder Metallalkoxides eines Metalles der Gruppen IVB, VB, VIB, VIIB und VIII des periodischen Systems, bei einer Temperatur von 0 bis 200° C und bei einem Molverhältnis Hydroperoxid: Amin von 50 : 1 bis 1 : 10.

2. Verfahren gemäss Anspruch 1, worin $E_1$, $E_2$, $E_3$ und $E_4$ Methyl sind.

3. Verfahren gemäss Anspruch 1, dadurch gekennzeichnet, dass man den Metall-Katalysator in einer Menge von 0,001 bis 0,1 Mol-%, berechnet auf das Hydroperoxid, verwendet.

4. Verfahren gemäss Anspruch 1, dadurch gekennzeichnet, dass die Reaktion bei 50 - 150° C ansgeführt wird.

5. Verfahren gemäss Anspruch 1, dadurch gekennzeichnet, dass das Molverhältnis Hydroperoxid: Amin 10 : 1 bis 1 : 1 beträgt.

6. Verfahren gemäss Anspruch 1, dadurch gekennzeichnet, dass als organisches Hydroperoxid tert-Butylhydroperoxid oder Cumol-hydroperoxid verwendet wird.

7. Verfahren gemäss Anspruch I, dadurch gekennzeichnet, dass als Katalysator Vanadyl-acetylacetonat, Cobalt-carbonyl, $Ti(OC_3H_7\text{-iso})_4$, $Mo(CO)_6$ oder $MoO_3$ verwendet wird.

8. Verfahren zur Herstellung von Verbindungen der Formel III,

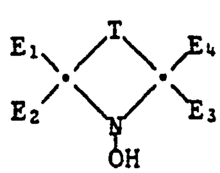

(III)

worin $E_1$, $E_2$, $E_3$, $E_4$ und T dieselbe Bedeutung wie in Anspruch 1 haben, durch Umsetzung einer Verbindung der Formel II gemäss Anspruch 1 und anschliessende reduktive Behandlung der Reaktionslösung ohne Isolierung des Zwischenproduktes der Formel I.

9. Verfahren gemäss Anspruch 8, dadurch gekennzeichnet, dass man die Reaktionslösung einer katalytischen Hydrierung in Gegenwart von Edelmetall- oder Nickel-Katalysatoren unterwirft.

## Claims

1. A process for the preparation of a nitroxyl of the formula I

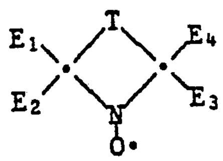

in which $E_1$ and $E_3$ are independently of one another $C_1$-$C_5$ alkyl or phenyl, $E_2$ and $E_4$ are independently of one another $C_1$-$_5$S alkyl or $E_1$ and $E_2$ together or $E_3$ and $E_4$ together are tetramethylene or pentamethylene, and T is a divalent group which, together with the nitrogen atom and the two tertiary carbon atoms, forms a 5- or 6-membered ring by reacting a cyclic secondary amine of the formula II

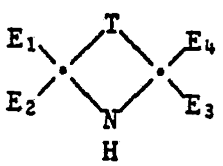

in an inert organic solvent, with an organic hydroperoxide in the presence of catalytic amounts of a metal carbonyl, a metal oxide, a metal acetylacetonate or a metal alkoxide of a metal from groups IVB, VB, VIB, VIIB and VIII of the periodic table, at a temperature of 0 to 200°C and at a molar ratio of hydroperoxide:amine of 50: 1 to 1 : 10.

2. A process according to claim 1, wherein $E_1$, $E_2$ $E_3$ and $E_4$ are methyl.

3. A process according to claim 1, wherein the metal catalyst is used in an amount of from 0.001 to 0.1 mol-%, based on the hydroperoxide.

4. A process according to claim 1, wherein the reaction is carried out at 50 - 150°C.

5. A process according to claim 1, wherein the molar ratio of hydroperoxide: amine is 10 : 1 to 1 : 1.

6. A process according to claim 1, wherein the organic hydroperoxide used is tert-butyl hydroperoxide or cumene hydroperoxide.

7. A process according to claim 1, wherein the catalyst used is vanadyl acetylacetonate, cobalt carbonyl, $Ti(OC_3H_7\text{-iso})_4$, $Mo(CO)_6$ or $MoO_3$.

8. A process for the preparation of compounds of the formula III

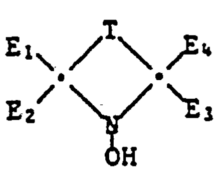

in which $E_1$, $E_2$, $E_3$, $E_4$ and T have the same meanings as in claim 1, by reacting a compound of the formula II according to claim 1 and subsequent reductive treatment of the reaction solution without isolation of the intermediate of the formula I.

9. A process according to claim 8, wherein the reaction solution is subjected to a catalytic hydrogenation in the presence of noble metal or nickel catalysts.

## Revendications

1. Procédé pour préparer des nitroxyles répondant à la formule I:

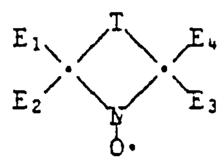

(I)

dans laquelle $E_1$ et $E_3$ représentent chacun, indépendamment l'un de l'autre, un alkyle en $E_1$-$C_5$ ou un phényle, $E_2$ et $E_4$ représentent chacun, indépendamment l'un de l'autre, un alkyle en $C_1$-$C_5$, ou $E_1$ et $E_2$ forment ensemble, ou $E_3$ et $E_4$ forment ensemble, un radical tétraméthylène ou pentaméthylène, et T représente un radical bivalent qui, avec l'atome d'azote et avec les deux atomes de carbone tertiaires, forme un cycle à 5 ou 6 maillons,

selon lequel on fait réagir une amine secondaire cyclique de formule II:

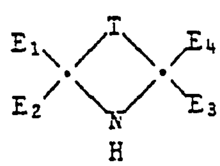

(II);

dans un solvant organique inerte, avec un hydroperoxyde organique, en présence de quantités catalytiques d'un métal-carbonyle, d'un oxyde de métal, d'un acétylacétonate de métal ou d'un alcoolate de métal dont le métal appartient à l'un des groupes IVB, VB, VIB, VIIB et VIII de la classification périodique, à une température de 0 à 200°C et avec un rapport molaire de l'hydroperoxyde à l'amine compris entre 50 : 1 et 1 : 10.

2. Procédé selon la revendication 1 dans lequel $E_1$, $E_2$, $E_3$ et $E_4$ représentent chacun un radical méthyle.

3. Procédé selon la revendication 1 caractérisé en ce qu'on utilise le catalyseur à base d'un métal en une quantité de 0,001 à 0,1 % en moles par rapport à l'hydroperoxyde.

4. Procédé selon la revendication 1 caractérisé en ce qu'on effectue la réaction à une température de 50 à 150°C.

5. Procédé selon la revendication 1 caractérisé en ce que le rapport molaire de l'hydroperoxyde

à l'amine est compris entre 10 : 1 et 1 : 1.

6. Procédé selon la revendication 1 caractérisé en ce qu'on utilise, comme hydroperoxyde organique, l'hydroperoxyde de tert-butyle ou l'hydroperoxyde du cumène.

7. Procédé selon la revendication 1 caractérisé en ce qu'on utilise, comme catalyseur, l'acétylacétonate de vanadyle, le cobalt-carbonyle, $Ti(OC_3H_7\text{-iso})_4$, $Mo(CO)_6$ ou $MoO_3$.

8. Procédé pour préparer des composés répondant à la formule III:

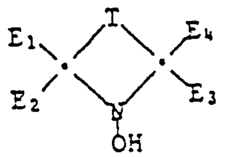

(III)

dans laquelle $E_1$, $E_2$, $E_3$, $E_4$ et T ont les mêmes significations que dans la revendication 1, procédé selon lequel on fait réagir selon la revendication 1 un composé da formule II puis, sans isoler le produit intermédiaire de formule I, on soumet la solution réactionnelle à un traitement réducteur.

9. Procédé selon la revendication 8 selon lequel on soumet la solution réactionnelle à une hydrogénation catalytique en présence d'un catalyseur à base d'un métal noble ou de nickel.